# EUROPÄISCHE PATENTSCHRIFT

(11) **EP 3 488 129 B1**
(45) Veröffentlichungstag und Bekanntmachung des Hinweises auf die Patenterteilung: **03.11.2021**
(21) Anmeldenummer: 17734034.6
(22) Anmeldetag: 22.06.2017
(51) Int. Cl.: F16K 11/044, G01N 9/26, F16K 27/02

(54) **VENTILVORRICHTUNG FÜR SCHALTANLAGEN ODER DERGLEICHEN SOWIE VERWENDUNGEN DERSELBEN**
VALVE DEVICE FOR SWITCHGEARS OR SIMILAR, AS WELL AS USES THEREOF
DISPOSITIF SOUPAPE POUR INSTALLATIONS DE DISTRIBUTION OU SIMILAIRES AINSI QU'UTILISATIONS DE CELLES-CI

(30) Priorität: 20.07.2016 DE 102016113339; 06.12.2016 DE 102016123588
(43) Veröffentlichungstag der Anmeldung: 29.05.2019
(73) Patentinhaber: Trafag AG, 8608 Bubikon (CH)
(72) Erfinder: HALBHEER, Remo, 8340 Hinwil (CH); ZEISEL, Dieter, 8127 Forch (CH)
(74) Vertreter: Kastel, Stefan
(86) Internationale Anmeldenummer: PCT/EP2017/065470
(87) Internationale Veröffentlichungsnummer: WO 2018/015101

(56) Entgegenhaltungen:
- CN-U- 201 531 646
- CN-U- 204 230 155
- CN-Y- 201 327 795
- GB-A- 347 172
- US-A- 5 313 839
- US-A1- 2005 072 479

## Beschreibung

Die Erfindung betrifft eine Überwachungsanordnung, die eine Überwachungsvorrichtung und eine Ventilvorrichtung zum Anschließen eines Dichtewächters oder dergleichen an eine hinsichtlich Gasdichte oder dergleichen zu überwachende Anlage umfasst. Außerdem betrifft die Erfindung eine mit einer solchen Überwachungsanordnung versehene elektrische Anlage. Weiter betrifft die Erfindung ein Überwachungsverfahren sowie eine Verwendung einer Ventilvorrichtung.

Die GB 347 172 A betrifft das Anschließen eines Druckanzeigegeräts an einen Luftreifen mittels einer Mehrwege-Ventilvorrichtung. Die US 2005/0072479 A1 betrifft eine Mehrwege-Ventilvorrichtung z.B. für Bewässerungssysteme. Eine weitere Mehrwege-Ventilvorrichtung ist aus der CN 201 531 646 U bekannt. Aus der US 5 313 839 A ist eine Ventilanordnung zum Anschließen eines Drucksensors an Klimaanlagen oder Kühlschränke bekannt.

Aus der DE 10 2010 055 249 A1 ist ein Dichtewächter zur Überwachung einer Gasdichte in einem Messvolumen mit einer Membran, die mit dem Messvolumen derart in Verbindung steht, dass sie sich bei Änderung einer Gasdichte in dem Messvolumen bewegt, und einer mit der Membran verbundenen Membranbewegungserfassungseinrichtung zum Umwandeln einer Membranbewegung in ein elektrisches Signal bekannt.

Zum technologischen Hintergrund zu Dichtewächtern wird insbesondere auf die JPS5578231A, die US 1527597 A, die US 3431785 A, die US 6125692 A, die US 2662394 A, die US 5421190 A und die DE 10232823 A verwiesen.

Dichtewächter sind Messgeräte zur Überwachung der Gasdichte eines zu überwachenden Gases. Wie aus der DE 10 2010 055 249 A1 bekannt, dienen Dichtewächter insbesondere zur Überwachung der Dichte in gasisolierten Hochund Mittelspannungsanlagen oder -geräten, wie zum Beispiel Hochspannungsschaltanlagen, -wandlern, -rohrleitungen, Schaltgeräten und Transformatoren, als Isolator befindlichen Gases, zum Beispiel SF6.

Es sind hierzu z.B. aus der DE 10232823 A1 auf elektronischen Messprinzipien basierende Dichtewächter bekannt, die mit einem elektronischen Dichtesensor als Messwertgeber versehen sind, der einen im Gas angeordneten Schwingquarz aufweist und als Messwert ein zur Dichte des Gases proportionales Frequenzsignal liefert, wobei das Frequenzsignal einer elektronischen Auswerteeinheit zugeführt wird.

Auf dem Markt haben sich dagegen auf mechanischen Messprinzipien beruhende Dichtewächter durchgesetzt, die aufgrund ihres mechanischen Messprinzips sehr zuverlässig und wartungsarm auch über sehr lange Zeiträume hinweg arbeiten. Im einfachsten und am häufigsten vorzutreffenden Fall steht hierbei eine über ein Referenzvolumen arbeitende Membran mit dem Messvolumen in Verbindung, wobei eine durch eine Änderung der Gasdichte verursachte Membranbewegung einen Schalter betätigt. Hierzu ist beispielsweise bei dem aus der DE 10 2010 055 249 A1 bekannten Dichtewächter eine Membran eines Metallbalgs an einen Schalter angeschlossen, so dass eine Membranbewegung über einen Mindestweg hinaus einen Schaltvorgang auslöst.

Aus der DE 27 51 206 A1 ist ein Dichtewächter mit einer Schalteinrichtung bekannt, die einen Quecksilberschalter aufweist.

Aus der DE 27 44 549 A1 ist eine Einrichtung zur Überwachung der Dichte eines Gases bekannt, das ein Manometer mit einem Zeiger und eine Rohrfeder als Ausdehnungselement und ein Kompensationselement zwischen dem Zeiger und dem Ausdehnungselement aufweist. Zum Antreiben des Zeigers ist ein Segmenthebel vorgesehen.

Aus der DD 282 076 A5 ist ein Dichtewächter zur Überwachung der Dichte eines Gases in einem hermetisch abgeschlossenen Behälter bekannt, mit dem eine möglichst hohe wartungsfreie Betriebsdauer auch bei kleineren Undichtigkeiten des Behälters ermöglicht werden. Hierzu sind zwei Metallbalge vorgesehen. Zwischen den Metallbalgen ist ein Stößel vorgesehen, der über einen Mitnehmer und einen Schalthebel einen als Doppelschalter ausgebildeten Schalter betätigt. Ein Schließen eines ersten Schaltkontakts wird ein Vorwarnsignal abgegeben, bei weiterem Dichteverlust wird ein zweiter Schaltkontakt geschlossen, mit dem ein Magnetventil betätigt wird, um Gas so lange in den Behälter nachzufüllen, bis die Kontakte wieder geöffnet werden.

Die CN 201327795Y und CN204230155 U betreffen abhängig von der SF₆-Dichte schaltende Schalter mit Einfüllstutzen für SF₆-Gas und der Möglichkeit des Anschlusses eines Geräts zur Schaltpunktüberprüfung.

Die Dichtewächter werden insbesondere eingesetzt, um Gasräume, die mit für die Umgebung bedenklichen Gasen gefüllt sind, insbesondere auf Leckagen zu überwachen. Daher sollten die Dichtewächter sehr zuverlässig arbeiten und sollten - auch über längere Zeiträume hinweg - zuverlässig Warnsignale bei Auftreten von bedenklichen Gasdichteänderungen liefern.

Aufgabe der Erfindung ist es, eine einfache Möglichkeit zur weiteren Verbesserung der Zuverlässigkeit einer Überwachung eines mit einem Schadgas befüllten Gasraums zur Verfügung zu stellen.

Diese Aufgabe wird durch eine Überwachungsanordnung nach Anspruch 1 gelöst. Vorteilhafte Ausgestaltungen sind Gegenstand der Unteransprüche. Die Überwachungsanordnung weist eine Überwachungseinrichtung mit einem Dichtewächter auf. Eine Kombination der Überwachungsanordnung mit einer zu überwachenden Anlage, ein damit durchführbares Überwachungsverfahren sowie eine Verwendung einer Mehrwege-Ventilvorrichtung für eine solche Überwachung sind in den Nebenansprüchen wiedergegeben.

Gemäß einem ersten Aspekt betrifft die Erfindung eine Überwachungsanordnung, die eine Überwachungsvorrichtung und ein Prüfgerät aufweist. Die Überwachungsvorrichtung weist die Überwachungseinrichtung und ein Mehrwege-Ventilvorrichtung auf, die in gemeinsamen Gehäuse mit der Überwachungseinrichtung integriert ist. Die Überwachungseinrichtung ist zum Überwachen wenigstens eines Gasparameters an eine ein Schadgas enthaltende und hinsichtlich des Gasparameters zu überwachende elektrische Anlage ausgebildet. Die Mehrwege-Ventilvorrichtung weist einen ersten Ventilweg, der als Gasanschluss zum Anschließen der Ventilvorrichtung an die Anlage ausgebildet ist, einen zweiten Ventilweg, der für die Gasüberleitung zu der Überwachungseinrichtung ausgebildet ist, einen dritten Ventilweg, der für eine Gasüberleitung zu einem Prüfgerät zur Überprüfung wenigstens einer Funktion der Überwachungseinrichtung ausgebildet ist, und eine automatische Umschalteinrichtung zum wahlweisen automatischen Verbinden des zweiten Ventilwegs an den ersten Ventilweg oder den dritten Ventilweg auf.

Die Überwachungseinrichtung weist einen Dichtewächter auf. Die Überwachungseinrichtung kann auch einen Drucksensor aufweisen. Bei einer bevorzugten Ausgestaltung ist ein Dichtewächter vorgesehen, der wahlweise oder kombiniert sowohl die Gasdichte als auch den Druck des zu überwachenden Gases erfasst.

Erfindungsgemäß weist die Überwachungsvorrichtung ein aus mehreren Teilen gebildetes Gesamtgehäuse auf, welches die Übertragungseinrichtung und die Mehrwege-Ventilvorrichtung beherbergt. Der Dichtewächter ist in einem Dichtewächtergehäuseteil des Gesamtgehäuses untergebracht und über ein rohrförmiges Anschlussgehäuseteil des Gesamtgehäuses an den zu überwachenden Gasraum anschließbar oder angeschlossen. In diesem Anschlussgehäuseteil ist die Mehrwege-Ventilvorrichtung untergebracht.

Es ist bevorzugt, dass die Umschalteinrichtung einen Ventilstößel aufweist, welcher in eine erste Schaltstellung, in der der dritte Gasanschluss geschlossen ist und der erste Gasanschluss mit dem zweiten Ventilweg verbunden ist, vorgespannt ist und durch Anschluss des Prüfgeräts an den dritten Gasanschluss in eine zweite Schaltstellung überführbar ist, in der der erste Gasanschluss geschlossen und der zweite Ventilweg mit dem dritten Gasanschluss verbunden ist.

Es ist bevorzugt, dass der Ventilstößel einen Vorsprung oder Stift umfasst, der bei Anschluss des Prüfgeräts durch ein Anschlusselement des Prüfgeräts erfasst wird und entgegen seiner Vorspannung aus der ersten Schaltstellung in die zweite Schaltstellung gedrückt wird.

Es ist bevorzugt, dass wenigstens eine Sensoreinrichtung zur Erfassung wenigstens eines Parameters, der ausgewählt ist aus der Gruppe von Parametern, die Temperatur, Dichte, Druck, Feuchte, Art des Gases und Vorhandensein eines Zersetzungsprodukts enthält, mit dem ersten Gasanschluss und/oder dem zweiten Ventilweg in Fluidverbindung steht.

Es ist bevorzugt, dass der erste Ventilweg als erster Gasanschluss ausgebildet ist.

Es ist bevorzugt, dass der dritte Ventilweg als zweiter Gasanschluss zum Anschließen des Prüfgeräts ausgebildet ist.

Erfindungsgemäß umfasst die Überwachungsvorrichtung die Mehrwege-Ventilvorrichtung und die Überwachungseinrichtung zur Überwachung wenigstens eines Gasparameters eines Schadgases in einer elektrischen Anlage.

Erfindungsgemäß ist vorgesehen, dass die Überwachungseinrichtung und die Mehrwege-Ventilvorrichtung in einem gemeinsamen Gehäuse integriert sind.

Es ist bevorzugt, dass die Überwachungseinrichtung eine Sensoreinrichtung aufweist.

Der Dichtewächter ist in einem Dichtewächtergehäuseteil eines Gesamtgehäuses der Überwachungsvorrichtung untergebracht und über ein rohrförmiges Anschlussgehäuseteil des Gesamtgehäuses an den zu überwachenden Gasraum anschließbar oder angeschlossen, wobei in diesem Anschlussgehäuseteil die Mehrwege-Ventilvorrichtung untergebracht ist.

Es ist bevorzugt, dass der zweite Ventilweg in dem Gesamtgehäuse untergebracht ist und zur Überleitung des Gases zu der in dem Gesamtgehäuse integrierten Überwachungseinrichtung dient.

Die erfindungsgemäße Überwachungsanordnung umfasst eine derartige Überwachungsvorrichtung nach einer der voranstehenden Ausgestaltungen und das Prüfgerät zum Überprüfen wenigstens einer Funktion der Überwachungseinrichtung.

Es ist erfindungsgemäß weiter vorgesehen, dass das Prüfgerät an den als zweiter Gasanschluss ausgebildeten dritten Ventilweg anschließbar oder angeschlossen ist.

Gemäß einem weiteren Aspekt schafft die Erfindung eine elektrische Anlage, umfassend einen mit einem Schadgas befüllten Gasraum und eine Überwachungsanordnung nach einer der voranstehenden Ausgestaltungen.

Gemäß einem weiteren Aspekt schafft die Erfindung ein Überwachungsverfahren zur Überwachung wenigstens eines Gasparameters eines Schadgases in einer elektrischen Anlage mittels einer Überwachungsvorrichtung, die eine Mehrwege-Ventilvorrichtung und eine Überwachungseinrichtung zur Überwachung des wenigstens einen Gasparameters eines Schadgases in einer elektrischen Anlage umfasst, umfassend die Schritte:
Anschließen der Überwachungseinrichtung mittels der Mehrwege-Ventilvorrichtung an die das Schadgas enthaltende und hinsichtlich des Gasparameters zu überwachende elektrische Anlage, wobei die Ventilvorrichtung über einen ersten Ventilweg als Gasanschluss an die Anlage angeschlossen wird und eine Gasüberleitung zu der Überwachungseinrichtung über einen zweiten Ventilweg der Ventilvorrichtung erfolgt, und wobei ein dritter Ventilweg der Ventilvorrichtung für eine Gasüberleitung zu einem Prüfgerät zur Überprüfung wenigstens einer Funktion der Überwachungseinrichtung verwendet wird und das Prüfgerät an den als dritter Gasanschluss ausgebildeten dritten Ventilweg angeschlossen wird, und wahlweises automatisches Verbinden des zweiten Ventilwegs an den ersten Ventilweg oder den dritten Ventilweg mittels einer automatischen Umschalteinrichtung der Ventilvorrichtung.

Gemäß einem weiteren Aspekt betrifft die Erfindung eine Verwendung einer in einer Überwachungsvorrichtung zusammen mit einer Überwachungseinrichtung integrierten Mehrwege-Ventilvorrichtung zum Anschließen der Überwachungseinrichtung zum Überwachen wenigstens eines Gasparameters an eine ein Schadgas enthaltende und hinsichtlich des Gasparameters zu überwachende elektrische Anlage, wobei die Mehrwege-Ventilvorrichtung einen ersten Ventilweg, der als Gasanschluss zum Anschließen der Ventilvorrichtung an die Anlage verwendet wird, einen zweiten Ventilweg, der für die Gasüberleitung zu der Überwachungseinrichtung verwendet wird, einen dritten Ventilweg, der für eine Gasüberleitung zu einem Prüfgerät zur Überprüfung wenigstens einer Funktion der Überwachungseinrichtung verwendet wird, und eine automatische Umschalteinrichtung umfasst, die zum wahlweisen automatischen Verbinden des zweiten Ventilwegs an den ersten Ventilweg oder den dritten Ventilweg verwendet wird.

Auch bei dem Verfahren und der Verwendung weist die Überwachungsvorrichtung ein aus mehreren Teilen gebildetes Gesamtgehäuse auf, welches die Übertragungseinrichtung und die Mehrwege-Ventilvorrichtung beherbergt. Der Dichtewächter ist in einem Dichtewächtergehäuseteil des Gesamtgehäuses untergebracht und wird über ein rohrförmiges Anschlussgehäuseteil des Gesamtgehäuses an den zu überwachenden Gasraum angeschlossen. In diesem Anschlussgehäuseteil ist die Mehrwege-Ventilvorrichtung untergebracht.

Bei einer Ausgestaltung der erfindungsgemäße Überwachungsanordnung ist eine in die Überwachungsvorrichtung integrierte Mehrwege-Ventilvorrichtung vorgesehen zum Anschließen der Überwachungsvorrichtung zum Überwachen wenigstens eines Gasparameters an eine ein Schadgas enthaltende hinsichtlich des Gasparameters zu überwachende Anlage, wobei die Mehrwege-Ventilvorrichtung einen ersten Gasanschluss zum Anschließen der Ventilvorrichtung an die Anlage, einen dritten Gasanschluss zum Anschließen eines Prüfgeräts zur Überprüfung der Funktion der Überwachungsvorrichtung und eine Umschalteinrichtung zum automatischen wahlweisen Verbinden des zweiten Gasanschlusses an den ersten oder den dritten Gasanschluss umfasst.

In bevorzugter Ausgestaltung betrifft die Erfindung eine Überwachungsanordnung mit einem Dichtewächter und einem an dem Dichtewächter integriert vorgesehene Mehrwege-Ventilvorrichtung zum Anschließen des Dichtewächters an eine hinsichtlich Gasdichte zu überwachende Anlage, wobei die Mehrwege-Ventilvorrichtung einen ersten Gasanschluss zum Anschließen der Ventilvorrichtung an die Anlage, einen Ventilweg zum Überleiten des Gases an den Dichtewächter, einen zweiten Gasanschluss zum Anschließen eines Prüfgeräts zur Überprüfung der Funktion des Dichtewächters und eine Umschalteinrichtung zum automatischen wahlweisen Verbinden des zweiten Ventilweges an den ersten oder den zweiten Gasanschluss.

Es ist bevorzugt, dass die Umschalteinrichtung eine Ventileinrichtung, insbesondere einen Ventilstößel, aufweist, welche in eine erste Schaltstellung, in der der zweite Gasanschluss geschlossen ist und der erste Gasanschluss mit dem zweiten Ventilweg verbunden ist, vorgespannt ist und durch Anschluss des Prüfgeräts an den zweiten Gasanschluss in eine zweite Schaltstellung überführbar ist, in der der erste Gasanschluss geschlossen und der zweite Ventilweg mit dem zweiten Gasanschluss verbunden ist.

Vorzugsweise umfasst die Rückschlagventilanordnung einen in seine Schließstellung vorgespannten Rückschlagventil-Öffner, der bei Anschluss der Überwachungsvorrichtung durch ein Anschlusselement der Überwachungsvorrichtung erfasst wird, um den Öffner entgegen seiner Vorspannung durch das Anschließen in seine Öffnungsstellung zu bewegen.

Vorzugsweise umfasst die Ventileinrichtung, insbesondere der Ventilstößel, einen Vorsprung oder Stift, der bei Anschluss des Prüfgeräts durch ein Anschlusselement des Prüfgeräts erfasst wird und entgegen seiner Vorspannung aus der ersten Schaltstellung in die zweite Schaltstellung gedrückt wird.

Es ist bevorzugt, dass wenigstens eine Sensoreinrichtung zur Erfassung wenigstens eines Parameters, der ausgewählt ist aus der Gruppe von Parametern, die Temperatur, Feuchte, Art des Gases und Vorhandensein eines Zersetzungsprodukts enthält, mit dem ersten Gasanschluss und/oder dem zweiten Ventilweg in Fluidverbindung steht.

Insbesondere schafft die Erfindung eine Überwachungsanordnung, insbesondere eine Dichteüberwachungsanordnung, umfassend eine Überwachungsvorrichtung, insbesondere eine Dichteüberwachungsvorrichtung, mit einem Gasparametersensor, insbesondere einem Dichtewächter, und eine Mehrwege-Ventilvorrichtung nach einem der voranstehenden Ausgestaltungen und eine in die Überwachungsvorrichtung integrierte Mehrwege-Ventilvorrichtung, wobei die Überwachungsvorrichtung mittels der Mehrwege-Ventilvorrichtung anschließbar ist.

Eine bevorzugte Ausgestaltung der Überwachungsanordnung umfasst ein an den zweiten Gasanschluss anzuschließendes Prüfgerät zum Überprüfen eines Schaltpunkts und/oder einer Funktion der Überwachungsvorrichtung, insbesondere des Dichtewächters.

Gemäß einem weiteren Aspekt schafft die Erfindung eine elektrische Anlage, insbesondere Schaltanlage, umfassend einen mit einem Gas, dessen Dichte zu überwachen ist, befüllten Gasraum und eine in einen Dichtewächter und/oder Überwachungsvorrichtung integrierte Mehrwege-Ventilvorrichtung nach einer der zuvor erläuterten Ausgestaltungen zum Anschließen des Dichtewächters und/oder der Überwachungsvorrichtung an den Gasraum.

Die Erfindung schafft insbesondere eine einfache Möglichkeit, die Funktion von Gasüberwachungseinrichtungen von Zeit zu Zeit zu überprüfen. Insbesondere ist es im Fall von an elektrischen Anlagen wie Schaltanlagen montierten Dichtewächtern erwünscht, dass der Zustand resp. die Schaltpunkte des Dichtewächters jederzeit ohne Demontage der selbigen überprüft werden können. Gleiches gilt für Dichtesensoren oder allgemein Sensoren, wie sie für die Überwachung des Anlagenzustandes eingesetzt werden (z.B. Feuchtesensoren, Sensoren, die die chemische Zersetzung des Isolationsgases SF6 messen z.B. SF4, WF6, SOF4, SO2F2, SOF2, SO2, und /oder HF; aber auch Öle und Staub erfassen).

Gemäß einer bevorzugten Ausgestaltung der Erfindung wird hierzu ein integriertes 3-Wege-Ventil vorgeschlagen, das sich vorzugsweise Tankflanschen verschiedener Hersteller anpasst.

Eine bevorzugte Ausgestaltung der Erfindung betrifft eine Verwendung eines integrierten automatischen 3-Wege-Ventils an SF6-Dichtewächtern.

Bei einer bevorzugten Ausgestaltung der Erfindung ist ein in einer Überwachungsvorrichtung integriertes 3-Wege-Ventil zur Nutzung an elektrischen Schaltanlagen vorgesehen, das
(1) im eingebauten Zustand eine Funktionskontrolle des Sensors / Wächters zulässt und/oder
(2) zusätzliche Sensorik enthält (z.B. Feuchte, Konzentration, Lichtbogenüberwachung, Zersetzungsprodukte oder dergleichen), die vorzugsweise gleichfalls überprüft werden kann.

Eine bevorzugte Verwendung der Erfindung oder deren Ausgestaltungen betrifft den Fall eines an einer elektrischen Schaltanlage montierten Dichtewächters. Ein Vorteil bevorzugter Ausgestaltungen ist, dass der Zustand resp. die Schaltpunkte des Wächters jederzeit ohne Demontage der selbigen überprüft werden können. Gleiches gilt für Dichtesensoren oder allgemein Sensoren wie sie für die Überwachung des Anlagenzustandes eingesetzt werden. Beispiele hierzu sind Feuchtesensoren, Sensoren, die die chemische Zersetzung des Isolationsgases SF6 messen z.B. SF4, WF6, SOF4, SO2F2, SOF2, SO2, und /oder HF; aber auch Öle und Staub. Auch wenn die Ausgestaltungen der Erfindung an Beispiel eines für SF6 besonders geeigneten Dichtewächters beschrieben werden, so können bei anderen Anwendungen auch andere Gase, insbesondere Isolationsgase überwacht werden und die Überwachungseinrichtung überprüft werden.

Bei einer Ausgestaltung wird ein automatisches 3-Wege-Ventil, welches direkt in den Dichtewächter integriert wird, vorgeschlagen, um eine Überprüfung des Dichtewächters ohne Demontage zu ermöglichen.

Eine besonders bevorzugte Ausgestaltung der Überwachungsvorrichtung betrifft einen Dichtewächter mit integriertem automatischen 3-Wege-Ventil zur Nutzung an elektrischen Schaltanlagen, das im eingebauten Zustand eine Funktionskontrolle des Wächters zulässt.

Ein Ausführungsbeispiel wird im Folgenden anhand der beigefügten Zeichnungen näher erläutert. Darin zeigt:

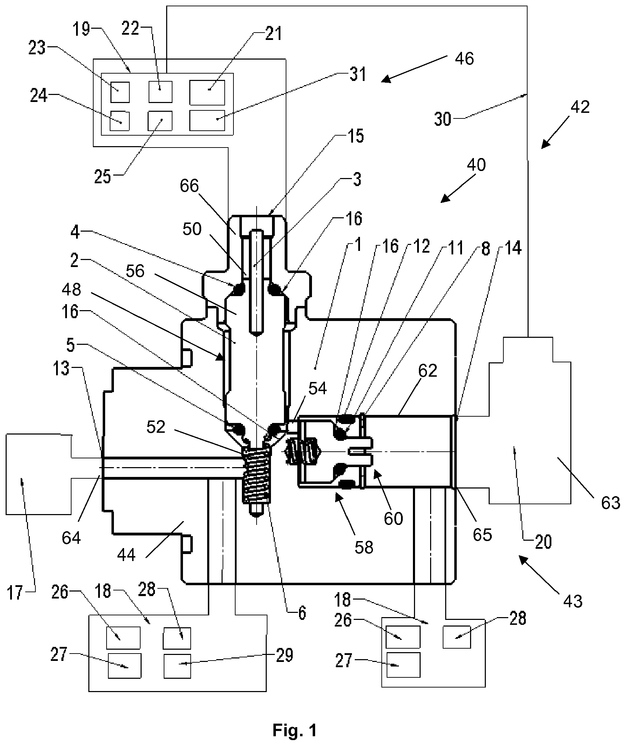

- Fig. 1: eine teils geschnittene Darstellung eines nicht zur Erfindung gehörenden Vergleichsbeispiels einer Überwachungsanordnung zur Überwachung des Gasraumes einer elektrischen Hochspannung- oder Mittelspannungsanlage, wie insbesondere Schaltanlage, wobei der Gasraum mit einem Isoliergas oder Schutzgas befüllt ist, wobei die Überwachungsanordnung eine Überwachungseinrichtung oder ein Überwachungsgerät, insbesondere einen Dichtewächter, zum Überwachen wenigstens eines Gasparameters des Isoliergas oder Schutzgases und eine Mehrwege-Ventilvorrichtung zum Anschließen der Überwachungseinrichtung an den Gasraum und zum wahlweisen Anschließen der Überwachungseinrichtung an ein Prüfgerät aufweist.
- Fig. 2: ein vergrößertes Detail der Mehrwege-Ventilvorrichtung im Bereich eines Gasanschlusses zum Anschließen des Prüfgeräts.
- Fig. 3: ein vergrößertes Detail der Mehrwege-Ventilvorrichtung im Bereich eines Gasanschlusses zum Anschließen der Überwachungseinrichtung.
- Fig. 4: eine schematische Darstellung der Schaltanlage mit der Überwachungseinrichtung.

- Fig. 5: eine Darstellung einer erfindungsgemäßen Ausführungsform einer Überwachungsvorrichtung zur Überwachung des Gasraums einer elektrischen Hochspannung- oder Mittelspannungsanlage, mit einer integrierten Mehrwege-Ventilvorrichtung.
- Fig. 6: ein vergrößertes Detail der Mehrwege-Ventilvorrichtung im Bereich eines Ventilwegs zum Anschließen eines Prüfgeräts.
- Fig. 7: eine erste Seitenansicht der Überwachungsvorrichtung von Fig. 5.
- Fig. 8: eine Vorderansicht der Überwachungsvorrichtung von Fig. 5.
- Fig. 9: eine weitere Seitenansicht der Überwachungsvorrichtung von Fig. 5.
- Fig. 10: eine perspektivische Darstellung der Überwachungsvorrichtung von Fig. 5.

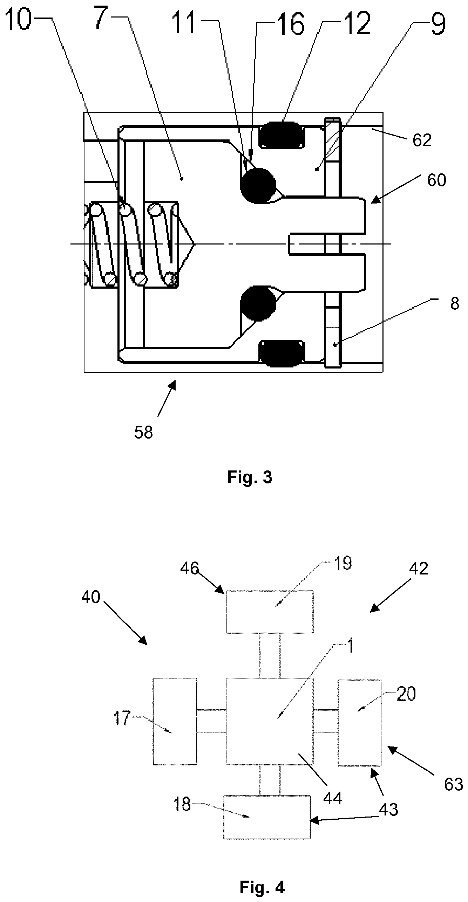

Im Folgenden werden Ausführungsbeispiele für eine elektrische Anlage 40 mit einem mit einem Isoliergas befüllten Gasraum 17 und einer Überwachungsanordnung 42 anhand der Figuren näher erläutert. Dabei wird zunächst auf Fig. 4 Bezug genommen, die einen schematischen Überblick zeigt.

Die elektrische Anlage 40 ist insbesondere eine gasisolierte Mittelspannungsanlage oder Hochspannungsanlage, wie zum Beispiel eine Schaltanlage, ein Spannungswandler, eine Hoch- oder Mittelspannungsrohrleitung, ein Schaltgerät oder ein Transformator. Die die Hochspannung oder Mittelspannung führenden Leitungen sind innerhalb eines geschlossenen Gasraumes 17 untergebracht, der ein Isoliergas, wie insbesondere SF6 oder eine Isoliergasmischung, z.B. mit SF6 und anderen Gasbestandteilen, enthält.

An den Gasraum 17 ist eine Überwachungsanordnung 42 zur Überwachung des Gasraumes 17, insbesondere zur Überwachung der Gasdichte oder zur Überwachung der Gasdichte und weiterer Parameter, wie insbesondere Temperatur, Feuchtigkeit, Gaszusammensetzung, Druck usw. angeschlossen. Die Überwachungsanordnung 42 hat hierzu eine Überwachungsvorrichtung 43. Die Überwachungsvorrichtung 43 hat eine Überwachungseinrichtung 63 und eine Mehrwege-Ventilvorrichtung 44 zum Anschließen der Überwachungseinrichtung 63 an den Gasraum 17.

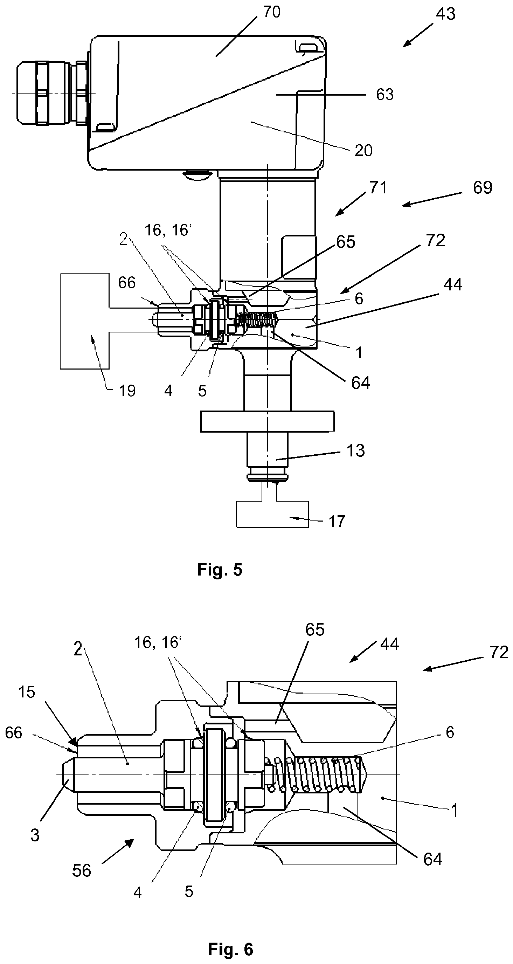

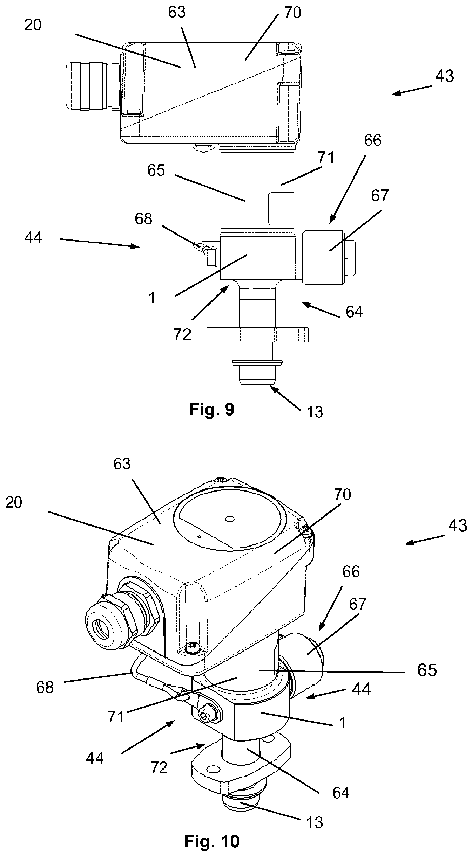

Bei dem in den Fig. 1 bis 3 dargestellten Vergleichsbeispiel der Überwachungsvorrichtung 43 sind die Überwachungseinrichtung 63 und die Mehrwege-Ventilvorrichtung 44 getrennt ausgeführt. Bei der in den Fig. 5 bis 10 dargestellten erfindungsgemäßen Ausführungsform der Überwachungsvorrichtung 43 sind die Mehrwege-Ventilvorrichtung 44 und die Überwachungseinrichtung 63 in einer Einheit integriert.

Bei beiden Ausgestaltungen ist die Mehrwege-Ventilvorrichtung 44 zum Anschließen der Überwachungseinrichtung 63, die zum Überwachen wenigstens eines Gasparameters ausgebildet ist, an eine ein Schadgas enthaltende und hinsichtlich des Gasparameters zu überwachende elektrische Anlage 40 ausgebildet. Die Mehrwege-Ventilvorrichtung 44 umfasst jeweils einen ersten Ventilweg 64 zum Anschließen der Ventilvorrichtung 44 an die Anlage 40, einen zweiten Ventilweg 65, der für die Gasüberleitung zu der Überwachungseinrichtung 43 ausgebildet ist, einen dritten Ventilweg 66, der für eine Gasüberleitung zu einem Prüfgerät 19 zur Überprüfung wenigstens einer Funktion der Überwachungseinrichtung 63 ausgebildet ist, und eine automatische Umschalteinrichtung 56 zum wahlweisen automatischen Verbinden des zweiten Ventilwegs 65 an den ersten Ventilweg 64 oder den dritten Ventilweg 66. Im Folgenden wird anhand der Fig. 1 bis 4 das Vergleichsbeispiel näher erläutert.

Die Mehrwege-Ventilvorrichtung 44 des Vergleichsbeispiels hat einen ersten Gasanschluss 13 zum Anschließen des Gasraums 17, einen dritten Gasanschluss 14 zum Anschließen der Überwachungsvorrichtung 43 und einen zweiten Gasanschluss 15 zum Anschließen einer Prüfanlage 46, mittels der wenigstens eine Funktion der Überwachungsvorrichtung 43 überprüfbar ist. Der erste Gasanschluss 13 ist an dem ersten Ventilweg 64 ausgebildet, der dritte Gasanschluss 14 ist an dem zweiten Ventilweg 65 ausgebildet, und der zweite Gasanschluss 15 ist an dem dritten Ventilweg 66 ausgebildet. Die Gasanschlüsse 13, 14, 15 können zum Beispiel mit Gewinde zum Anschließen von entsprechenden Gasleitungen ausgebildet sein; selbstverständlich sind auch andere Gasanschlussformen denkbar.

Die Mehrwege-Ventilvorrichtung 44 ist zum automatischen Umschalten und Verbinden der Gasanschlüsse 13, 14, 15 ausgebildet. Dabei ist die Mehrwege-Ventilvorrichtung 44 derart ausgebildet, dass dann, wenn lediglich der Gasraum 17 an den ersten Gasanschluss 13 angeschlossen ist und kein Gerät an die anderen Gasanschlüsse 14, 15 angeschlossen ist, die anderen Gasanschlüsse 14 und 15 gesperrt sind.

Die Mehrwege-Ventilvorrichtung 44 ist derart ausgebildet, dass dann, wenn die Überwachungsvorrichtung 43 an den dritten Gasanschluss 14 angeschlossen ist, aber der zweite Gasanschluss 15 ungenutzt bleibt, der erste Gasanschluss 13 mit dem dritten Gasanschluss 14 in Fluidverbindung ist, so dass der Gasraum 17 mit der Überwachungsvorrichtung 43 in Fluidverbindung ist, so dass der Gasraum 17 mit der Überwachungsvorrichtung 43 überwacht werden kann.

Weiter ist die Mehrwege-Ventilvorrichtung 44 derart ausgebildet, dass beim Anschluss der Prüfanlage 46 an den zweiten Gasanschluss 15 ein automatisches Umschalten der Fluidverbindung zwischen den Gasanschlüssen 13, 14, 15 derart erfolgt, dass der erste Gasanschluss 13 gesperrt ist und der dritte Gasanschluss 14 mit dem zweiten Gasanschluss 15 verbunden ist, so dass die an den dritten Gasanschluss 14 angeschlossene Überwachungsvorrichtung 42 durch die Prüfanlage 46 auf wenigstens eine Funktion hin überprüft werden kann.

So kann die Prüfanlage 46 beispielsweise durch ein mobiles Prüfgerät 19 gebildet sein, das zur Überprüfung mehrerer derartiger Überwachungsvorrichtungen 43 je nach Bedarf an den jeweiligen zweiten Gasanschluss 15 angeschlossen werden kann, um so sofort ohne Ausbau oder sonstigen Aufwand und ohne Beeinträchtigung der Dichtheit oder Gassicherheit eine Überprüfung der Überwachungsvorrichtung 43 in deren laufenden Betrieb durchzuführen.

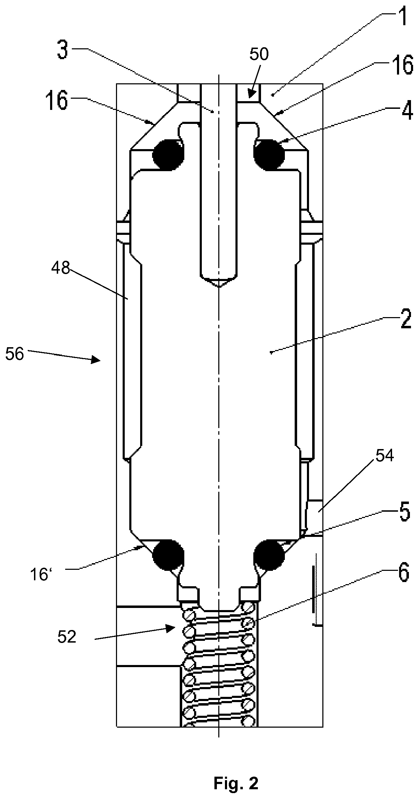

In Fig. 1 ist der Gesamtaufbau dargestellt, Fig. 2 zeigt den Aufbau der Mehrwege-Ventilvorrichtung 44 im Bereich des zweiten Gasanschlusses 15, und Fig. 3 zeigt den Aufbau der Mehrwege-Ventilvorrichtung 44 im Bereich des dritten Gasanschlusses 14.

Wie aus Fig. 1 ersichtlich, weist die Mehrwege-Ventilvorrichtung 44 ein Ventilgehäuse 1 auf, in dem die Gasanschlüsse 13, 14, 15, z.B. mit entsprechenden Anschlussgewinden (nicht dargestellt) eingebracht sind.

Der Gasraum 17 kann durch einen Tank unterschiedlicher Hersteller gebildet sein. Der erste Gasanschluss 13 kann, z.B. mit unterschiedlichen Adaptern (nicht dargestellt) an unterschiedliche Tankstutzen angeschlossen werden.

Im Bereich des zweiten Gasanschlusses 15 ist ein Ventilstößel 2 als Öffner und Schließer vorgesehen. Der Ventilstößel 2 ist mittels eines Fühlers, der den Anschluss einer Prüfanlage 46 erfasst, zwischen einer Schließstellung und einer Öffnungsstellung bewegbar.

Hierzu weist der Ventilstößel 2 insbesondere einen Zylinder oder Stift 3 für eine automatische Schließ- und Öffnungsfunktion derart auf, dass der Zylinder oder Stift 3 zu dem Endbereich des zweiten Gasanschlusses 15 vorragt und bei Anschluss der Prüfanlage 46 in das Ventilgehäuse 1 hineingedrückt wird, um so den Ventilstößel 2 aus seiner Schließposition in seine Öffnungsposition zu bewegen.

Wie sich insbesondere aus Fig. 2 ergibt, ist der Ventilstößel 2 in einem Fluidkanalraum 48 verschiebbar untergebracht. An beiden Längsenden des Fluidkanalraums 48 weist dieser jeweils einen Dichtkonus 16, 16' auf.

An dem in Fig. 2 oben dargestellten erste Ende, das dem Anschluss für die Prüfanlage 46 zugewandt ist, ist ein erster Durchlass 50 zur Prüfanlage 46 vorgesehen, der bei Bewegung des Ventilstößels 2 gegen den hier angeordneten ersten Dichtkonus 16 mittels einer ersten Dichtung 4 verschlossen wird.

An dem in Fig. 2 unten dargestellten zweiten Ende ist ein zweiter Durchlass 52 zu dem ersten Gasanschluss 13 vorgesehen, der bei Verfahren des Ventilstößels 2 gegen den zweiten Dichtkonus 16' mittels einer zweiten Dichtung 5 verschließbar ist. Zwischen dem Ventilstößel 2 und der Wandung des Fluidkanalraums 48 ist ein Abstand frei gelassen. In den so frei gelassenen Fluidkanalraum 48 mündet seitlich eine Passage 54 hin zu dem zweiten Gasanschluss 14, der näher in Fig. 3 dargestellt ist.

Der Ventilstößel 2 ist durch eine Druckfeder 6 in seine in Fig. 1 dargestellte Schließstellung vorgespannt. Fig. 2 zeigt die Öffnungsstellung des Ventilstößels 2 bei angeschlossener Prüfanlage 46.

In der Schließstellung des Ventilstößels 2 ist der erste Gasanschluss 13 über den zweiten Durchlass 52 an dem zweiten Ende des Fluidkanalraums 48, den Fluidkanalraum 48 und die seitliche Passage 54 mit dem dritten Gasanschluss 14 verbunden. Der Ventilstößel 2 erfüllt darüber hinaus noch eine Rückschlagventilfunktion, indem er durch einen Überdruck an dem ersten Gasanschluss 13 in seine Schließstellung gedrängt wird.

In der Öffnungsstellung des Ventilstößels 2 ist der zweite Durchlass 52 durch Anlage der zweiten Dichtung 5 an den zweiten Dichtkonus 16' verschlossen und der erste Durchlass 54 ist geöffnet, so dass der zweite Gasanschluss 15 über den ersten Durchlass 54, den Fluidkanalraum 48 und die Passage 54 mit dem dritten Gasanschluss 14 verbunden ist.

Somit ist durch den Ventilstößel 2 eine Umschalteinrichtung 56 gebildet, mit der bei Anschluss der Prüfanlage 46 ein automatisches Umschalten zwischen einer ersten Schaltstellung - der Schließstellung - und einer zweiten Schaltstellung - der Öffnungsstellung - erfolgt, um so im laufenden Betrieb die Überwachungsvorrichtung zur Überprüfung von dem Gasraum 17 abzukoppeln und an ein Prüfgerät 19 anzukoppeln.

Der dritte Gasanschluss 14 ist mit einer Rückschlagventilanordnung 58 versehen, die näher in Fig. 3 dargestellt ist. Die Rückschlagventilanordnung 58 weist eine Schließstellung, in der der zweite Gasanschluss 14 geschlossen ist, und eine Öffnungsstellung, in der der zweite Gasanschluss 14 geöffnet ist, und einen Öffnungsmechanismus 60 auf, der einen Anschluss der Überwachungsvorrichtung 43 an den dritten Gasanschluss 14 erfasst und auf den Anschluss hin automatisch die Rückschlagventilanordnung 58 aus der Schließstellung in die Öffnungsstellung überführt.

Die Rückschlagventilanordnung 58 ist derart ausgebildet, dass sie bei Überdruck im Inneren des Ventilgehäuses 1 und insbesondere bei Überdruck auf Seiten des ersten Gasanschlusses 13 in seine Schließstellung gedrängt wird. Weiter ist die Rückschlagventilanordnung 58 darüber hinaus noch in ihre Schließstellung, z.B. durch eine Druckfeder 10, vorgespannt.

Wie aus Fig. 3 ersichtlich weist die Rückschlagventilanordnung 58 einen in einer Kanalöffnung 62 verschiebbar angeordneten Öffner 7 zum Öffnen und Schließen des Rückschlagventils am Anschluss für die Überwachungsvorrichtung 42 auf. Weiter ist in der Kanalöffnung 62 ein Sicherungsring 8 zum Sichern der Rückschlagventilbaugruppe am Anschluss für die Überwachungsvorrichtung 42 eingesetzt. Die Rückschlagventilanordnung 58 weist weiter eine Hülse 9 mit einem Dichtkonus 16 auf, an dem eine an dem Öffner 7 angebrachte Dichtung 11 abdichten kann. Die Hülse 9 ist außen mit einer Dichtung 12 gegen die Wandung der Kanalöffnung 62 abgedichtet. Der Öffner 7 ist mit der Druckfeder 10 in seine Schließstellung gegen den Dichtkonus 16 der Hülse 9 vorgespannt.

Wie in Fig. 1 dargestellt, weist die Mehrwege-Ventilvorrichtung 44 in bevorzugter Ausgestaltung einen Sensoreinbau 18 auf. In Fig. 1 sind zwei mögliche Anschlüsse für den Sensoreinbau 18 angedeutet. In einer ersten Version ist der Sensoreinbau 18 mit dem ersten Gasanschluss 13 verbunden und ist somit permanent und/oder direkt mit dem Gasraum 17 verbunden. Bei einer zweiten Ausgestaltung ist der Sensoreinbau 18 mit dem dritten Gasanschluss 14 verbunden. Die erste Ausgestaltung hat den Vorteil des direkten Anschlusses. So können Temperatur, Feuchte, Gaszusammensetzung unmittelbarer gemessen werden; auch ist die Erfassung eines Lichtbogens möglich. Die zweite Ausgestaltung hat den Vorteil, dass der Sensoreinbau 18 z.B. durch die Prüfanlage 46 ohne Ausbau überprüft werden kann. Der Sensoreinbau 18 kann so ein Ausführungsbeispiel für die Überwachungsvorrichtung 43 sein oder kann Teil der Überwachungsvorrichtung 43 sein.

Der Sensoreinbau 18 weist in bevorzugter Ausgestaltung wenigstens einen, mehrere oder alle der folgenden Sensoren auf: Feuchtesensor 26, Temperatursensor 27, Zersetzungsproduktsensor 28, Lichtbogenzähler 29 bzw. eine Lichtbogenerfassung und Drucksensor.

Wie in Fig. 1 angedeutet weist die Überwachungsvorrichtung wenigstens einen Dichtewächter 20 auf. Der Dichtewächter 20 ist insbesondere so ausgebildet, wie dies in der WO 2015/101442 A1 und der DE 10 2013 115 009 A1, auf die für weitere Einzelheiten ausdrücklich verwiesen wird, beschrieben und gezeigt ist.

Die Prüfanlage 46 weist in bevorzugter Ausgestaltung wenigstens ein Prüfgerät 19 zur Prüfung eines Schaltpunkts des Dichtewächters 20 und/oder zur Prüfung einer Funktion des Dichtewächters 20 und/oder eines Sensors 26, 27, 28 auf.

In bevorzugter Ausgestaltung weist das Prüfgerät 19 einen Referenzsensor und/oder einen Referenzdichtewächter 21 auf. Damit kann ein z.B. über eine Leitung geliefertes Signal 30 der Überwachungsvorrichtung 43 mit einem Referenzwert verglichen werden, um so die Funktion zu überprüfen.

In bevorzugter Ausgestaltung weist das Prüfgerät 19 wenigstens eine mehrere oder alle der folgenden Einheiten auf: eine Pumpe 20, eine Anzeige 23, z.B. ein Display, eine elektronische Steuerung 24, eine Benutzerschnittstelle 25, insbesondere in Form einer GUI und/oder einen Speicher und/oder Mikrokontroller 31.

Im Folgenden wird die Funktion der hier beschrieben Überwachungsanordnung 42 näher erläutert. Die Figuren zeigen insbesondere ein Ventil, welches wenigstens drei Anschlüsse 13; 14; 15 hat.

Der erste Gasanschluss 13 wird fix an den Gasraum 17, insbesondere einer Schaltanlage, angeschlossen. In diesem Zustand sind der dritte Gasanschluss 14 und der zweite Gasanschluss 15 geschlossen.

Durch das Anschließen eines Dichtewächters 20 an den dritten Gasanschluss 14 wird der Öffner 7 des Rückschlagventils geöffnet und die Gasdurchführung von dem Gasraum 17 zum Dichtewächter 20 ist ermöglicht.

Im Betrieb kann man über den für eine Prüfanlage 46 vorgesehenen zweiten Gasanschluss 15 ein Prüfgerät 19 anschließen. Durch das Anschließen des Prüfgeräts 19 koppelt sich der Gasraum 17 der Schaltanlage oder dergleichen mittels des Ventilstößels 2 von dem Dichtewächter 20 und dem Prüfgerät 19 automatisch ab. Im abgekoppelten Zustand der Schaltanlage ist die Durchführung nur zwischen Dichtewächter 20 - dritter Gasanschluss 14 - und dem Prüfgerät 19 - zweiter Gasanschluss 15 - gewährleistet. Das Prüfgerät 19 nutzt das eingeschlossene Rest-Gas aus dem Gasraum 17 der Schaltanlage, um den Dichtewächter 20 zu prüfen. Nach der Prüfung des Dichtewächters 20 wird das Rest-Gas durch das Prüfgerät 19 wieder zurückgepumpt. Mit dem Abkoppeln des Prüfgeräts 19 schaltet die Mehrwege-Ventilvorrichtung 44 mittels des Ventilstößels 2 automatisch wieder um.

Im Folgenden wird anhand der Fig. 5 bis 10 die erfindungsgemäße Ausführungsform der Überwachungsvorrichtung 43 näher erläutert.

Während bei einer denkbaren Verwendung des Vergleichsbeispiels ein separates 3-Wege-Ventil auf eine Schaltanlage zu montieren ist und auf diesem 3-Wege-Ventil eine Überwachungseinrichtung 63, wie insbesondere ein Dichtewächter 20 aufzusetzen ist, ist bei einer Verwendung der erfindungsgemäßen Ausführungsform vorgesehen, ein automatisches 3-Wege-Ventil direkt zusammen mit der Überwachungseinrichtung 63 in einer Einheit zu integrieren. Z.B. ist eine Mehrwege-Ventilvorrichtung 44 direkt in einen Dichtewächter 20 integriert.

Bei der folgenden Beschreibung der erfindungsgemäßen Ausführungsform werden für entsprechende Teile die gleichen Bezugszeichen wie bei dem Vergleichsbeispiel verwendet und es wird zu weiteren Einzelheiten und Funktionen dieser Teile ergänzend auf die obige Beschreibung des Vergleichsbeispiels verwiesen.

Wie aus den Fig. 5, 7 bis 10 ersichtlich weist die erfindungsgemäße Ausführungsform der Überwachungsvorrichtung 43 ein aus mehreren Teilen gebildetes Gesamtgehäuse 69 auf, welches die Überwachungseinrichtung 63 und die Mehrwege-Ventilvorrichtung 44 beherbergt.

Die Überwachungseinrichtung 63 weist in dem dargestellten Ausführungsbeispiel den Dichtewächter 20 in einem Dichtewächtergehäuseteil 70 auf. Über ein rohrartiges Anschlussgehäuseteil 71 wird die Überwachungseinrichtung 63 an den zu überwachenden Gasraum 17 angeschlossen. In diesem Anschlussgehäuseteil 71 ist die beispielsweise ein automatisches 3-Wege-Ventil aufweisende Mehrwege-Ventilvorrichtung 44 untergebracht.

Der erste Ventilweg 64 weist den ersten Gasanschluss 13 zum Anschließen des Gasraums 17 auf. Der zweite Ventilweg 65 ist in dem Gesamtgehäuse 69 untergebracht und dient zur Überleitung des Gases zu der in dem Gesamtgehäuse 69 integrierten Überwachungseinrichtung 63. Der zweite Ventilweg ist z.B. als Gasüberleitung von dem Anschlussgehäuseteil 71 in den Dichtewächtergehäuseteil 70 ausgebildet. Der dritte Ventilweg 66 weist in bevorzugter Ausgestaltung den zweiten Gasanschluss 15 zum Anschließen des Prüfgeräts 19 auf. Bei der dargestellten Ausgestaltung kann eine z.B. als Gewindedeckel ausgebildete Schutzkappe 67 aufweisen. Die Schutzkappe 67 kann mit einer Schutzkappensicherung 68 an einem der Gehäuseteile 70, 71 des Gesamtgehäuses 69 gesichert sein.

Die Fig. 5 und 6 zeigen einen Schnitt durch die Mehrwege-Ventilvorrichtung 44. Dabei dient das Anschlussgehäuseteil 71 als Ventilgehäuse, d.h. es weist einen Ventilgehäusebereich 72 auf, in dem der bewegliche Ventilstößel 2 untergebracht ist. Wie bei dem Vergleichsbeispiel sind der Stift 3 für die automatische Schließund Öffnungsfunktion, die Dichtung 4 für das Ventil Prüfanschluss und die Dichtung 5 für das Ventil zum Gasraum 17 bzw. zu einer Schaltanlage und die die Druckfeder 6 für das Ventil zum Prüfanschluss vorgesehen.

Fig. 6 zeigt den Ventilstößel 2 in seiner Normalposition, ohne dass das Prüfgerät 19 an den zweiten Gasanschluss (Prüfanschluss oder Wartungsanschluss) 15 angeschlossen ist. Die Druckfeder 6 drückt den Ventilstößel 2 in der Fig. 6 nach links, so dass die Dichtung 4 den dritten Ventilweg 66, der hier zu dem zweiten Gasanschluss 15 führt, verschließt. Es besteht eine Fluidverbindung zwischen dem ersten und dem zweiten Ventilweg 64, 65 und somit stehen die Überwachungseinrichtung 63 - insbesondere der Dichtewächter 20 - und der erste Gasanschluss 13 in Fluidverbindung. So kann z.B. der Dichtewächter den an den ersten Gasanschluss 13 angeschlossenen Gasraum 17 überwachen.

Wird das Prüfgerät 19 angeschlossen, dann wird über den Stift 3 der Ventilstößel 2 in Fig. 6 nach rechts verschoben, wodurch der zweite Ventilweg 65 geöffnet wird und der erste Ventilweg 64 mittels der Dichtung 5 verschlossen wird. So steht die Überwachungseinrichtung 63 mit dem Prüfgerät 19 in Fluidverbindung und die Verbindung zu dem überwachenden Gasraum 17 ist getrennt.

Die Fig. 5 bis 10 zeigen insbesondere einen Dichtewächter 20 mit einem integrierten Wartungsanschluss (zweiter Gasanschluss 15). Der erste Gasanschluss 13 wird fix am Gasraum 17 der Schaltanlage angeschlossen.

In dem gezeigten Normalbetriebszustand ist der zweite Gasanschluss 15 geschlossen, und der Durchfluss ist vom Gasraum 17 zum Dichtewächter 20 gewährleistet.

Im Betrieb kann man über den zweiten Gasanschluss 15 (Gasanschluss Prüfanlage) ein Prüfgerät 19 anschließen.

Durch das Anschließen eines Prüfgerätes 19 an den zweiten Gasanschluss 15 wird der Ventilstößel 2 hineingedrückt und öffnet durch diese Bewegung den Durchgang von der Prüfanlage 19 zum Dichtewächter 20. Gleichzeitig wird der Durchgang vom Gasraum 17 zum Dichtewächter 20 geschlossen. Das Prüfgerät 19 nutzt das eingeschlossene Rest-Gas der Schaltanlage, um den Dichtewächter 20 zu prüfen. Nach der Prüfung des Dichtewächters 20 wird das Rest-Gas durch das Prüfgerät 19 wieder zurückgepumpt. Mit dem Abkoppeln der Prüfeinheit (z.B. Prüfgerät 19) schaltet der Ventilstößel 2 automatisch wieder um.

Bei der in den Fig. 5 bis 10 gezeigten Ausgestaltung ist demnach eine Mehrwege-Ventilvorrichtung 44, welche direkt in den Dichtewächter 20 integriert ist, dargestellt, die eine Überprüfung des Dichtewächters 20 ohne Demontage ermöglicht.

Wie in Fig. 5 angedeutet, umfasst eine Überwachungsvorrichtung 43 eine Überwachungseinrichtung 63 sowie eine Mehrwege-Ventilvorrichtung 44. Die Überwachungseinrichtung 63 weist wenigstens einen Dichtewächter 20, welcher wenigstens einen integrierten dritten Ventilweg 66 aufweist. Der erste Ventilweg 64 wird fix am Gasraum der Schaltanlage 17 angeschlossen. In diesem Zustand ist der dritte Ventilweg 66 geschlossen und der Durchfluss ist vom Gasraum 17 zum Dichtewächter 20 gewährleistet. Durch die Konstruktion ist es möglich, während des laufenden Betriebs über den dritten Ventilweg 66 ein Prüfgerät 19 anzuschließen.

Fig. 6 zeigt einen Ausschnitt aus Fig. 5 und stellt ein Detail der Mehrwege-Ventilvorrichtung 44 dar. Durch Anschließen des Prüfgeräts 19 an den dritten Ventilweg 66 wird der Ventilstößel 2 entgegen der Federkraft der Druckfeder 6 hineingedrückt und öffnet somit den Durchgang von der Prüfanlage 19 zum Dichtewächter 20, wobei der Ventilstößel 2 mit der Dichtung 5 gegen den Dichtkonus 16' verfährt. Mit dem Abkoppeln der Prüfeinheit schaltet der Ventilstößel 2 durch Verfahren gegen den ersten Dichtkonus 16 automatisch wieder um.

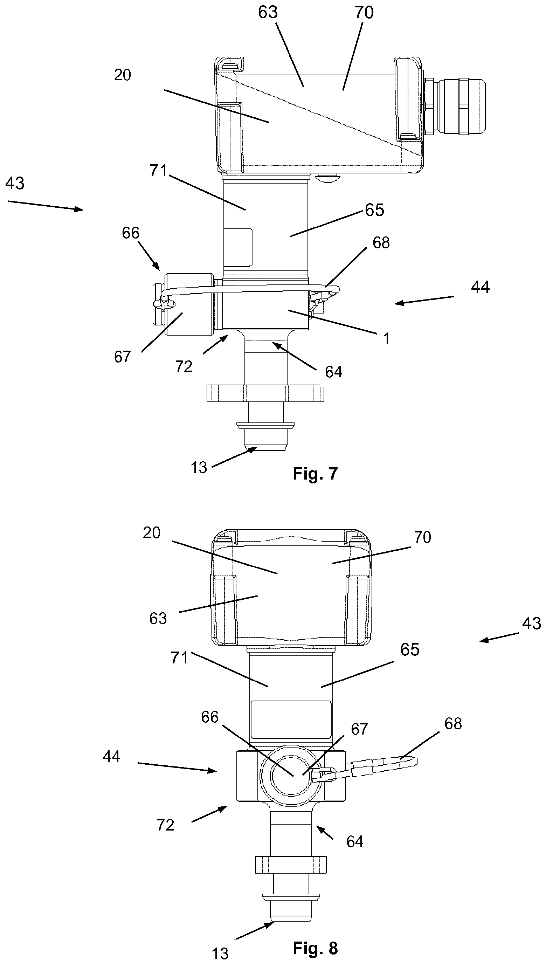

Fig. 7 bis 10 zeigen weitere Darstellungen der Überwachungsvorrichtung 43. Bei diesen Ausführungsformen ist der dritte Ventilweg 66 mit der Schutzkappe 67 mit Schutzkappensicherung 68 versehen, um Verschmutzungen des dritten Ventilwegs 66 zu verhindern.

### Bezugszeichenliste:

- 1: Ventilgehäuse
- 2: Ventilstößel: Öffner / Schließer
- 3: Zylinder Stift für automatische Schließ- / Öffnungs-Funktion
- 4: Dichtung Ventil Prüfanschluss
- 5: Dichtung Ventil Schaltanlage
- 6: Druckfeder für Ventil Prüfanschluss / Schaltanlage
- 7: Öffner Rückschlagventil Dichtewächteranschluss
- 8: Sicherungsring Rückschlagventil-Baugruppe Dichtewächteranschluss
- 9: Hülse Rückschlagventil Dichtewächteranschluss
- 10: Druckfeder für Rückschlagventil Dichtewächteranschluss
- 11: Dichtung für Rückschlagventil Dichtewächteranschluss
- 12: Dichtung Rückschlagventil-Baugruppe
- 13: erster Gasanschluss (insbesondere Schaltanlage)
- 14: dritter Gasanschluss (Überwachungsvorrichtung, insbesondere Dichtewächter)
- 15: zweiter Gasanschluss (Prüfanlage)
- 16: erster Dichtkonus
- 16': zweiter Dichtkonus
- 17: Gasraum der zu überwachenden Schaltanlage
- 18: Sensoreinbau
- 19: Prüfgerät zur Schaltpunkt- oder Funktionsprüfung
- 20: Dichtewächter
- 21: Referenzsensor oder Referenzdichtewächter
- 22: Pumpe
- 23: Anzeige (Display)
- 24: Elektronische Steuerung
- 25: GUI = Schnittstelle zum Benutzer
- 26: Feuchtesensor
- 27: Temperatursensor
- 28: Zersetzungsproduktsensor
- 29: Lichtbogenzähler, Lichtbogenerfassung
- 30: Signal des eingebauten Dichtewächters zum Prüfgerät
- 31: Speicher & Mikrokontroller
- 40: elektrische Anlage
- 42: Überwachungsanordnung
- 43: Überwachungsvorrichtung
- 44: Mehrwege-Ventilvorrichtung
- 46: Prüfanlage
- 48: Fluidkanalraum
- 50: erster Durchlass
- 52: zweiter Durchlass
- 54: Passage
- 56: Umschalteinrichtung
- 58: Rückschlag-Ventilanordnung
- 60: Öffnungsmechanismus
- 62: Kanalöffnung
- 63: Überwachungseinrichtung
- 64: erster Ventilweg
- 65: zweiter Ventilweg
- 66: dritter Ventilweg
- 67: Schutzkappe
- 68: Schutzkappensicherung
- 69: Gesamtgehäuse
- 70: Dichtewächtergehäuseteil
- 71: Anschlussgehäuseteil
- 72: Ventilgehäusebereich

## Patentansprüche

1. Überwachungsanordnung (42), umfassend
eine Überwachungsvorrichtung (43), die eine Überwachungseinrichtung (63) zur Überwachung wenigstens eines Gasparameters eines Schadgases in einer elektrischen Anlage (40) und eine Mehrwege-Ventilvorrichtung (44) umfasst, und ein Prüfgerät (19) zum Überprüfen wenigstens einer Funktion der Überwachungseinrichtung (43);
wobei die Mehrwege-Ventilvorrichtung (44) einen ersten Ventilweg (64), der als Gasanschluss (13) zum Anschließen der Ventilvorrichtung (44) an die Anlage (40) ausgebildet ist, einen zweiten Ventilweg (65), der für die Gasüberleitung zu der Überwachungseinrichtung (63) ausgebildet ist, einen dritten Ventilweg (66), der für eine Gasüberleitung zu dem Prüfgerät (19) zur Überprüfung wenigstens einer Funktion der Überwachungseinrichtung (63) ausgebildet ist, und eine automatische Umschalteinrichtung (56) zum wahlweisen automatischen Verbinden des zweiten Ventilwegs (65) an den ersten Ventilweg (64) oder den dritten Ventilweg (66) umfasst, wobei das Prüfgerät (19) an den als zweiter Gasanschluss (15) ausgebildeten dritten Ventilweg (66) anschließbar oder angeschlossen ist, wobei die Überwachungseinrichtung (63) wenigstens einen Dichtewächter (20) aufweist, **dadurch gekennzeichnet,**
**dass** die Überwachungsvorrichtung (43) ein aus mehreren Teilen gebildetes Gesamtgehäuse (69) aufweist, welches die Übertragungseinrichtung (63) und die Mehrwege-Ventilvorrichtung (44) beherbergt, und dass der Dichtewächter (20) in einem Dichtewächtergehäuseteil (70) des Gesamtgehäuses (69) untergebracht ist und über ein rohrförmiges Anschlussgehäuseteil (71) des Gesamtgehäuses (69) an den zu überwachenden Gasraum (17) anschließbar ist oder angeschlossen ist, wobei in diesem Anschlussgehäuseteil (71) die Mehrwege-Ventilvorrichtung (44) untergebracht ist.

2. Überwachungsanordnung (42) nach Anspruch 1, **dadurch gekennzeichnet, dass** die Umschalteinrichtung (56) einen Ventilstößel (2) aufweist, welcher in eine erste Schaltstellung, in der der zweite Gasanschluss (15) geschlossen ist und der erste Gasanschluss (13) mit dem zweiten Ventilweg (65) verbunden ist, vorgespannt ist und durch Anschluss des Prüfgeräts (19) an den zweiten Gasanschluss (15) in eine zweite Schaltstellung überführbar ist, in der der erste Gasanschluss (13) geschlossen und der zweite Ventilweg (65) mit dem zweiten Gasanschluss (15) verbunden ist.

3. Überwachungsanordnung (42) nach Anspruch 2,
**dadurch gekennzeichnet,**
**dass** der Ventilstößel (2) einen Vorsprung oder Stift (3) umfasst, der bei Anschluss des Prüfgeräts (19) durch ein Anschlusselement des Prüfgeräts (19) erfasst wird und entgegen seiner Vorspannung aus der ersten Schaltstellung in die zweite Schaltstellung gedrückt wird.

4. Überwachungsanordnung (42) nach einem der voranstehenden Ansprüche, **dadurch gekennzeichnet,**
**dass** wenigstens eine Sensoreinrichtung (18) zur Erfassung wenigstens eines Parameters, der ausgewählt ist aus der Gruppe von Parametern, die Temperatur, Feuchte, Art des Gases und Vorhandensein eines Zersetzungsprodukts enthält, mit dem ersten Gasanschluss (13) und/oder dem zweiten Ventilweg (65) in Fluidverbindung steht.

5. Überwachungsanordnung (42) nach einem der voranstehenden Ansprüche, **dadurch gekennzeichnet,**
**dass** die Überwachungseinrichtung (63) eine Sensoreinrichtung (18) aufweist.

6. Überwachungsanordnung (42) nach einem der voranstehenden Ansprüche, **dadurch gekennzeichnet,**
**dass** der zweite Ventilweg (65) als Gasüberleitung von dem Anschlussgehäuseteil (71) in den Dichtewächtergehäuseteil (70) ausgebildet ist.

7. Elektrische Anlage (40), umfassend einen mit einem Schadgas befüllten Gasraum (17) und eine Überwachungsanordnung (42) nach einem der voranstehenden Ansprüche.

8. Überwachungsverfahren zur Überwachung wenigstens eines Gasparameters eines Schadgases in einer elektrischen Anlage (40) mittels einer Überwachungsvorrichtung (43), die eine Überwachungseinrichtung (63) zur Überwachung des wenigstens einen Gasparameters, welche Überwachungseinrichtung (63) wenigstens einen Dichtewächter (20) aufweist, und eine Mehrwege-Ventilvorrichtung (44) und ein aus mehreren Teilen gebildetes Gesamtgehäuse (69) umfasst, welches die Übertragungseinrichtung (63) und die Mehrwege-Ventilvorrichtung (44) beherbergt, wobei der Dichtewächter (20) in einem Dichtewächtergehäuseteil (70) des Gesamtgehäuses (69) untergebracht ist und die Mehrwege-Ventilvorrichtung (44) in einem rohrförmigen Anschlussgehäuseteil (71) des Gesamtgehäuses (69) untergebracht ist, umfassend die Schritte:
Anschließen der Überwachungseinrichtung (63) und somit des Dichtewächters (20) mittels des rohrförmigen Anschlussgehäuseteils (71) und der darin untergebrachten Mehrwege-Ventilvorrichtung (44) an die das Schadgas enthaltende und hinsichtlich des Gasparameters zu überwachende elektrische Anlage (40), wobei die Mehrwege-Ventilvorrichtung (44) über einen ersten Ventilweg (64) als Gasanschluss (13) an die Anlage (40) angeschlossen wird, wobei eine Gasüberleitung zu der Überwachungseinrichtung (63) über einen zweiten Ventilweg (65) der Ventilvorrichtung (44) erfolgt, und wobei ein dritter Ventilweg (66) der Ventilvorrichtung (44) für eine Gasüberleitung zu einem Prüfgerät (19) zur Überprüfung wenigstens einer Funktion der Überwachungseinrichtung (63) verwendet wird und das Prüfgerät (19) an den als zweiter Gasanschluss (15) ausgebildeten dritten Ventilweg (66) angeschlossen wird, und
wahlweises automatisches Verbinden des zweiten Ventilwegs (65) an den ersten Ventilweg (64) oder den dritten Ventilweg (66) mittels einer automatischen Umschalteinrichtung (56) der Ventilvorrichtung (44).

9. Verwendung einer in einem Gesamtgehäuse (69) einer Überwachungsvorrichtung (42) integrierten Mehrwege-Ventilvorrichtung (44) zum Anschließen einer Überwachungseinrichtung (63) der Überwachungsvorrichtung (42) zum Überwachen wenigstens eines Gasparameters an eine ein Schadgas enthaltende und hinsichtlich des Gasparameters zu überwachende elektrische Anlage (40), wobei die Überwachungseinrichtung (63) einen Dichtewächter (20) aufweist und die Überwachungsvorrichtung (43) das aus mehreren Teilen gebildete Gesamtgehäuse (69) aufweist, welches die Übertragungseinrichtung (63) und die Mehrwege-Ventilvorrichtung (44) beherbergt, und wobei der Dichtewächter (20) in einem Dichtewächtergehäuseteil (70) des Gesamtgehäuses (69) untergebracht ist und über ein rohrförmiges Anschlussgehäuseteil (71) des Gesamtgehäuses (69 an den zu überwachenden Gasraum (17) anschließbar ist oder angeschlossen ist, wobei in diesem Anschlussgehäuseteil (71) die Mehrwege-Ventilvorrichtung (44) untergebracht ist, wobei die Mehrwege-Ventilvorrichtung (44) einen ersten Ventilweg (64), der als Gasanschluss (13) zum Anschließen der Ventilvorrichtung (44) an die Anlage (40) verwendet wird, einen zweiten Ventilweg (65), der für die Gasüberleitung zu der Überwachungseinrichtung (63) verwendet wird, einen dritten Ventilweg (66), der für eine Gasüberleitung zu einem Prüfgerät (19) zur Überprüfung wenigstens einer Funktion der Überwachungseinrichtung (63) verwendet wird, und eine automatische Umschalteinrichtung (56) umfasst, die zum wahlweisen automatischen Verbinden des zweiten Ventilwegs (65) an den ersten Ventilweg (64) oder den dritten Ventilweg (66) verwendet wird.

## Claims

1. Monitoring arrangement (42) comprising:
a monitoring device (43) comprising monitoring means (63) for monitoring at least one gas parameter of a pollutant gas in an electrical system (40) and a multiway valve device (44), and a testing device (19) for checking at least one function of the monitoring means (43);
the multiway valve device (44) comprising a first valve path (64) formed as a gas connection (13) for connecting the valve device (44) to the system (40), a second valve path (65) formed for gas transfer to the monitoring means (63), a third valve path (66) formed for gas transfer to the testing device (19) for checking at least one function of the monitoring means (63) and an automatic switching device (56) for selectively automatically connecting the second valve path (65) to the first valve path (64) or to the third valve path (66), the testing device (19) being connectable or connected to the third valve path (66), which is designed as a second gas connection (15), the monitoring means (63) having at least one density monitor (20),
**characterized in that**
the monitoring device (43) has an overall housing (69) which is formed from a plurality of parts and accommodates the monitoring means (63) and the multiway valve device (44) and **in that** the density monitor (20) is accommodated in a density monitor housing part (70) of the overall housing (69) and can be connected or is connected to the gas space (17) to be monitored via a tubular connection housing part (71) of the overall housing (69),
the multiway valve device (44) being accommodated in this connection housing part (71).

2. Monitoring arrangement (42) according to claim 1,
**characterized in that**
the changeover device (56) has a valve tappet (2) which is biased into a first switching position, in which the second gas connection (15) is closed and the first gas connection (13) is connected to the second valve path (65) and can be transferred into a second switching position, in which the first gas connection (13) is closed and the second valve path (65) is connected to the second gas connection (15), by connecting the testing device (19) to the second gas connection (15).

3. Monitoring arrangement (42) according to claim 2,
**characterized in that**
the valve tappet (2) comprises a projection or pin (3) which, when the testing device (19) is connected, is gripped by a connecting element of the testing device (19) and is pressed out of the first switching position into the second switching position counter to its bias.

4. Monitoring arrangement (42) according to any of the preceding claims, **characterized in that**
at least one sensor device (18) for detecting at least one parameter selected from the group of parameters including temperature, humidity, type of gas and presence of a decomposition product is in fluid communication with the first gas connection (13) and/or the second valve path (65).

5. Monitoring arrangement (42) according to any of the preceding claims, **characterized in that**
the monitoring device (63) comprises a sensor device (18).

6. Monitoring arrangement (42) according to one of the preceding claims, **characterized in**
**that** the second valve path (65) is formed as a gas transfer from the connection housing part (71) into the density monitor housing part (70).

7. Electrical system (40) comprising a gas space (17) filled with a pollutant gas and a monitoring arrangement (42) according to any of the preceding claims.

8. Monitoring method for monitoring at least one gas parameter of a pollutant gas in an electrical system (40) by means of a monitoring device (43) comprising a monitoring means (63) for monitoring the at least one gas parameter, which monitoring means (63) has at least one density monitor (20), and a multiway valve device (44) and an overall housing (69) formed from a plurality of parts, housing the monitoring means (63) and the multiway valve device (44), wherein the density monitor (20) is housed in a density monitor housing part (70) of the overall housing (69) and the multiway valve device (44) is housed in a tubular connection housing part (71) of the overall housing (69), the method comprising the steps of:
connecting the monitoring means (63) and thus the density monitor (20) to the electrical system (40) containing the pollutant gas and to be monitored with regard to the gas parameter, by means of the tubular connection housing (71) and the multiway valve device (44) accommodated therein, the multiway valve device (44) being connected to the system (40) via a first valve path (64) as gas connection (13), wherein a gas transfer to the monitoring means (63) is effected via a second valve path (65) of the valve device (44), and wherein a third valve path (66) of the valve device (44) is used for a gas transfer to a testing device (19) for checking at least one function of the monitoring means (63), and the testing device (19) is connected to the third valve path (66) formed as a second gas connection (15), and
optionally automatically connecting the second valve path (65) to the first valve path (64) or the third valve path (66) by means of an automatic changeover device (56) of the valve device (44).

9. Use of a multiway valve device (44) integrated in an overall housing (69) of a monitoring arrangement (42) for connecting a monitoring means (63) of the monitoring arrangement (42) for monitoring at least one gas parameter to an electrical system (40) containing a pollutant gas and to be monitored with regard to the gas, the monitoring means (63) having a density monitor (20) and the monitoring device (43) having the overall housing (69) formed from a plurality of parts, which accommodates the monitoring means (63) and the multiway valve device (44), and wherein the density monitor (20) is accommodated in a density monitor housing part (70) of the overall housing (69) and can be connected or is connected to the gas space (17) to be monitored via a tubular connection housing part (71) of the overall housing (69), said connection housing part (71) accommodating said multiway valve device (44), said multi-way valve device (44) having a first valve path (64) used as a gas connection (13) for connecting said valve device (44) to said system (40), a second valve path (65) used for gas transfer to said monitoring means (63), and a third valve path (66) used for gas transfer to a testing device (19) for checking at least one function of the monitoring means (63), and automatic switching means (56) used for selectively automatically connecting the second valve path (65) to the first valve path (64) or the third valve path (66).

## Revendications

1. Arrangement de surveillance (42) comprenant:
un dispositif de surveillance (43), qui comprend un moyen de surveillance (63) pour surveiller au moins un paramètre de gaz d'un gaz polluant dans une installation électrique (40) et un agencement de soupape à plusieurs voies (44), et un dispositif de test (19) pour vérifier au moins une fonction du dispositif de surveillance (43);
l'agencement de soupape à plusieurs voies (44) comprenant une première voie de soupape (64) formée comme raccord de gaz (13) pour connecter l'agencement de soupape (44) à l'installation (40), une deuxième voie de soupape (65) formée pour le transfert de gaz au moyen de surveillance (63),
une troisième voie de soupape (66) formée pour le transfert de gaz vers le dispositif de test (19) pour vérifier au moins une fonction du moyen de surveillance (63) et un dispositif de commutation automatique (56) pour la connexion automatique sélective de la deuxième voie de soupape (65) à la première voie de soupape (64) ou à la troisième voie de soupape (66), le dispositif de test (19) pouvant être connecté ou étant connecté à la troisième voie de soupape (66), qui est conçue comme un deuxième raccord de gaz (15), le moyen de surveillance (63) présentant au moins un moniteur de densité (20),
**caractérisé en ce**
**que** le dispositif de surveillance (43) présente un boîtier global (69) formé de plusieurs pièces, dans lequel sont logés le moyen de surveillance (63) et
l'agencement de soupape à plusieurs voies (44) et en ce que le moniteur de densité (20) est logé dans une partie de boîtier de moniteur de densité (70) du boîtier global (69) et peut être raccordé ou est raccordé à l'espace de gaz (17) à surveiller par l'intermédiaire d'une partie de boîtier de raccordement tubulaire (71) du boîtier global (69), le dispositif de soupape à plusieurs voies (44) étant logé dans cette partie de boîtier de raccordement (71).

2. Arrangement de surveillance (42) selon la revendication 1,
**caractérisé en ce**
**que** le dispositif de commutation (56) présente un poussoir de soupape (2) qui est précontraint dans une première position de commutation, dans laquelle le deuxième raccord de gaz (15) est fermé et le premier raccord de gaz (13) est reliée à la deuxième voie de soupape (65), et qui peut être amené dans une deuxième position de commutation, dans laquelle le premier raccord de gaz (13) est fermé et la deuxième voie de soupape (65) est reliée au deuxième raccord de gaz (15), par le raccordement du dispositif de test (19) au deuxième raccord de gaz (15).

3. Arrangement de surveillance (42) selon la revendication 2,
**caractérisé en ce**
**que** le poussoir de soupape (2) comprend une saillie ou une goupille (3) qui, lorsque le dispositif de test (19) est connecté, est saisie par un élément de connexion du dispositif de test (19) et est pressée de la première position de commutation à la deuxième position de commutation contre sa précontrainte.

4. Arrangement de surveillance (42) selon l'une quelconque des revendications précédentes,
**caractérisé en ce**
**qu'**au moins un dispositif capteur (18) pour détecter au moins un paramètre sélectionné dans le groupe de paramètres comprenant la température, l'humidité, le type de gaz et la présence d'un produit de décomposition est en communication fluidique avec le premier raccord de gaz (13) et/ou le second voie de soupape (65).

5. Arrangement de surveillance (42) selon l'une quelconque des revendications précédentes,
**caractérisé en ce**
**que** le moyen de surveillance (63) comprend un dispositif capteur (18).

6. Arrangement de surveillance (42) selon l'une quelconque des revendications précédentes,
**caractérisé en ce**
**que** la deuxième voie de soupape (65) est formée comme un transfert de gaz de la partie de boîtier de raccordement (71) dans la partie de boîtier de moniteur de densité (70).

7. Installation électrique (40) comprenant un espace de gaz (17) remplie d'un gaz polluant et un agencement de surveillance (42) selon l'une quelconque des revendications précédentes.

8. Procédé de surveillance d'au moins un paramètre de gaz d'un gaz polluant dans un installation électrique (40) au moyen d'un dispositif de surveillance (43) qui comprend un moyen de surveillance (63) pour surveiller l'au moins un paramètre de gaz, lequel moyen de surveillance (63) présente au moins un moniteur de densité (20), et un agencement de soupape à plusieurs voies (44) et un boîtier global (69) formé de plusieurs pièces, qui loge le moyen de surveillance (63) et l'agencement de soupape à plusieurs voies (44), dans lequel le moniteur de densité (20) est logé dans une partie de boîtier de moniteur de densité (70) du boîtier global (69) et le dispositif de soupape à plusieurs voies (44) est logé dans une partie de boîtier de raccordement tubulaire (71) du boîtier global (69), comprenant les étapes:
le raccordement du moyen de surveillance (63) et donc du moniteur de densité (20) a l'installation électrique (40) contenant le gaz polluant et devant être surveillé en ce qui concerne le paramètre de gaz, au moyen de la partie de boîtier de raccordement tubulaire (71) et de l'agencement de soupape à plusieurs voies (44) logé dans celui-ci, l'agencement de soupape à plusieurs voies (44) étant relié à l'installation (40) par une première voie de soupape (64) en tant que raccordement de gaz (13), dans lequel un transfert de gaz vers le moyen de surveillance (63) est effectué par l'intermédiaire d'une deuxième voie de soupape (65) de l'agencement de soupape (44), et dans lequel une troisième voie de soupape (66) de l'agencement de soupape (44) est utilisée pour un transfert de gaz vers un dispositif de test (19) pour vérifier au moins une fonction du moyen de surveillance (63), et le dispositif de test (19) est relié à la troisième voie de soupape (66) formée comme un deuxième raccord de gaz (15), et
en option, la connexion automatique de la deuxième voie de soupape (65) à la première voie de soupape (64) ou à la troisième voie de soupape (66) au moyen d'un dispositif de commutation automatique (56) de l'agencement de soupape (44).

9. Utilisation d'un agencement de soupape à plusieurs voies (44) intégré dans un boîtier global (69) d'un arrangement de surveillance (42) pour raccorder un moyen de surveillance (63) de l'arrangement de surveillance (42) destiné à surveiller au moins un paramètre de gaz à un installation électrique (40) contenant un gaz polluant et devant être surveillée en ce qui concerne le paramètre de gaz, dans lequel le moyen de surveillance (63) comprend un moniteur de densité (20) et le dispositif de surveillance (43) comprend le boîtier global (69) formé de plusieurs parties, qui loge le moyen de surveillance (63) et le dispositif de soupape à plusieurs voies (44) et dans lequel le moniteur de densité (20) est logé dans une partie de boîtier de moniteur de densité (70) du boîtier global (69) et est ou peut être relié à l'espace de gaz (17) à surveiller par l'intermédiaire d'une partie de boîtier de raccordement tubulaire (71) du boîtier global (69), dans lequel l'agencement de soupape à plusieurs voies (44) est logé dans cette partie de boîtier de raccordement (71), dans lequel l'agencement de soupape à plusieurs voies (44) présente une première voie de soupape (64), utilisé comme raccord de gaz (13) pour relier l'agencement de soupape (44) au installation (40), un deuxième voie de soupape (65) utilisé pour le transfert de gaz vers le moyen de surveillance (63), un troisième voie de soupape (66) utilisé pour le transfert de gaz vers un dispositif de test (19) pour vérifier au moins une fonction du moyen de surveillance (63), et un dispositif de commutation automatique (56) utilisé pour la connexion automatique sélective de la deuxième voie de soupape (65) à la première voie de soupape (64) ou à la troisième voie de soupape (66).
